# EUROPEAN PATENT APPLICATION

(11) **EP 1 967 583 A1**
(43) Date of publication of application: **10.09.2008**
(21) Application number: 06841745.0
(22) Date of filing: 18.12.2006
(51) Int. Cl.: C12N 9/24, C12N 15/56

(54) **NOVEL FRUCTOFURANOSIDASE ACTIVITY FOR OBTAINING THE PREBIOTIC OLIGOSACCHARIDE 6-KESTOSE**

(30) Priority: 26.12.2005 ES 200503195
(71) Applicant: UNIVERSIDAD AUTÓNOMA DE MADRID, 28049 Madrid (ES); CONSEJO SUPERIOR DE INVESTIGACIONES, 28006 Madrid (ES)
(72) Inventor: FERNANDEZ LOBATO, María, 28760 Tres Cantos, Madrid (ES); ALVARO BENITO, Miguel, 40005 Segovia (ES); DE ABREU FELIPE, Miguel Antonio, 28049 Madrid (ES); FERNANDEZ ARROJO, Lucía, 28700 San Sebastian de los Reyes, Madrid (ES); PLOU GASCA, Francisco José, 28049 Madrid (ES)
(74) Representative: Carvajal y Urquijo, Isabel
(86) International application number: PCT/ES2006/000693
(87) International publication number: WO 2007/074187

(57) **Abstract**

The invention relates to a novel fructofuranosidase activity for obtaining the prebiotic oligosaccharide 6-kestose. One object of the invention is to characterize a novel transfructosylase activity which is associated with the extracellular invertase of *Schwanniomyces occidentalis* (specifically the strains selected from the group consisting of ATCC260077, ATCC7410 and ATCC20499) and which can be used to obtain prebiotic oligosaccharides, mainly 6-kestose, which are widely used in the food industry. Another object of the invention relates to the method for obtaining an enzymatic product as well as to the substantially pure enzyme with fructofuranosidase activity.

SEQUENCE LISTING

<110>  Universidad Autónoma de Madrid y CSIC

<120>  NOVEL FRUCTOFURANOSIDASE ACTIVITY FOR OBTAINING THE PREBIOTIC
OLIGOSACCHARIDE 6-KESTOSE

<130>  P-INCI-N-05-0228

<160>  4

<170>  PatentIn version 3.3

<210>  1
<211>  535
<212>  PRT
<213>  Debaryomyces occidentalis

<400>  1

Met Val Gln Val Leu Ser Val Leu Val Ile Pro Leu Leu Thr Leu Phe
1               5                   10                  15

Phe Gly Tyr Val Ala Ser Ser Ser Ile Asp Leu Ser Val Asp Thr Ser
            20                  25                  30

Glu Tyr Asn Arg Pro Leu Ile His Phe Thr Pro Glu Lys Gly Trp Met
        35                  40                  45

Asn Asp Pro Asn Gly Leu Phe Tyr Asp Lys Thr Ala Lys Leu Trp His
    50                  55                  60

Leu Tyr Phe Gln Tyr Asn Pro Asn Ala Thr Ala Trp Gly Gln Pro Leu
65                  70                  75                  80

Tyr Trp Gly His Ala Thr Ser Asn Asp Leu Val His Trp Asp Glu His
                85                  90                  95

Glu Ile Ala Ile Gly Pro Glu His Asp Asn Glu Gly Ile Phe Ser Gly
            100                 105                 110

Ser Ile Val Val Asp His Asn Asn Thr Ser Gly Phe Phe Asn Ser Ser
        115                 120                 125

Ile Asp Pro Asn Gln Arg Ile Val Ala Ile Tyr Thr Asn Asn Ile Pro
    130                 135                 140

Asp Leu Gln Thr Gln Asp Ile Ala Phe Ser Leu Asp Gly Gly Tyr Thr
145                 150                 155                 160

Phe Thr Lys Tyr Glu Asn Asn Pro Val Ile Asp Val Ser Ser Asn Gln
                165                 170                 175

Phe Arg Asp Pro Lys Val Phe Trp His Glu Asp Ser Asn Gln Trp Ile
            180                 185                 190

Met Val Val Leu Lys Ser Gln Glu Tyr Lys Ile Gln Ile Phe Gly Ser
        195                 200                 205

Ala Asn Leu Lys Asn Trp Val Leu Asn Ser Asn Phe Ser Ser Gly Tyr
    210                 215                 220

Tyr Gly Asn Gln Tyr Glu Cys Pro Gly Leu Ile Glu Val Pro Ile Glu
225                 230                 235                 240

Asn Ser Asp Lys Ser Lys Trp Val Met Phe Leu Ala Ile Asn Pro Gly
                245                 250                 255

Ser Pro Leu Gly Gly Ser Ile Asn Gln Tyr Phe Val Gly Asp Phe Asp
            260                 265                 270

Gly Phe Gln Phe Val Pro Asp Asp Ser Gln Thr Arg Phe Val Asp Ile
        275                 280                 285

Gly Lys Asp Phe Tyr Ala Phe Gln Thr Phe Ser Glu Val Glu His Gly
    290                 295                 300

Val Leu Gly Leu Ala Trp Ala Ser Asn Trp Gln Tyr Ala Asp Gln Val
305                 310                 315                 320

Pro Thr Asn Pro Trp Arg Ser Ser Thr Ser Leu Ala Arg Asn Tyr Thr
                325                 330                 335

Leu Arg Tyr Val His Thr Asn Ala Glu Thr Lys Gln Leu Thr Leu Ile
            340                 345                 350

Gln Asn Pro Val Leu Pro Asp Ser Ile Asn Val Val Asp Lys Leu Lys
        355                 360                 365

Lys Lys Asn Val Lys Leu Thr Asn Lys Lys Pro Ile Lys Thr Asn Phe
    370                 375                 380

Lys Gly Ser Thr Gly Leu Phe Asp Phe Asn Ile Thr Phe Lys Val Leu
385                 390                 395                 400

Asn Leu Asn Val Ser Pro Gly Lys Thr His Phe Asp Ile Leu Ile Asn
                405                 410                 415

Ser Gln Glu Leu Asn Ser Ser Val Asp Ser Ile Lys Ile Gly Phe Asp
            420                 425                 430

Ser Ser Gln Ser Ser Phe Tyr Ile Asp Arg His Ile Pro Asn Val Glu
        435                 440                 445

Phe Pro Arg Lys Gln Phe Phe Thr Asp Lys Leu Ala Ala Tyr Leu Glu
    450                 455                 460

Pro Leu Asp Tyr Asp Gln Asp Leu Arg Val Phe Ser Leu Tyr Gly Ile
465                 470                 475                 480

Val Asp Lys Asn Ile Ile Glu Leu Tyr Phe Asn Asp Gly Thr Val Ala
                485                 490                 495

Met Thr Asn Thr Phe Phe Met Gly Glu Gly Lys Tyr Pro His Asp Ile
            500                 505                 510

Gln Ile Val Thr Asp Thr Glu Glu Pro Leu Phe Glu Leu Glu Ser Val
        515                 520                 525

Ile Ile Arg Glu Leu Asn Lys
    530                 535

<210>  2
<211>  30
<212>  DNA
<213>  Artificial Sequence

<220>
<223>  primer

<400>  2
cgggatccat ggtacaagtt ttaagtgtat                                        30

<210>  3
<211>  30
<212>  DNA
<213>  Artificial Sequence

<220>
<223>  primer

<400>  3
cctcgagcta cttatttagt tctctaatga                                        30

<210>  4
<211>  1608
<212>  DNA
<213>  Debaryomyces occidentalis

<400>  4
atggtacaag ttttaagtgt attagtgatt cctttgctaa ccctattttt tgggtatgtg      60

gcttcgtcct cgattgactt atcggtagat acgtcagagt ataaccggcc attaattcat     120

tttactccgg aaaaaggatg gatgaatgat ccgaatggtc tattctacga taaaactgct     180

aagctttggc acttatactt ccagtataat ccaaatgcta ctgcgtgggg gcaaccatta     240

tattggggac acgctacgtc gaatgatttg gtacattggg atgaacatga gatagctatt     300

ggacctgaac acgataatga aggtatcttt tcaggtagta ttgtcgttga ccataataat     360

acctctggtt tcttcaatag ctcaattgat ccaaaccaaa gaattgttgc catttatacc     420

aacaatatac cagatttaca aacccaagac attgcatttt cgttagatgg aggatatact     480

tttactaaat atgagaataa tcctgtgatt gatgtctcct caaaccaatt ccgtgatcca     540

aaagttttct ggcatgaaga ttcaaatcaa tggatcatgg ttgttctgaa atcgcaagag 600
tacaaaattc aaatttttgg ttcagcaaat ttgaagaact gggttttgaa ttcaaatttt 660
tcttctggtt attacggaaa tcagtatgaa tgtccaggtt taattgaagt tcctattgag 720
aattcagaca aatcaaagtg ggttatgttt ttagcaatta atccgggatc gcctttgggt 780
ggttcgatta accaatattt tgttggtgat tttgacggct tccagtttgt tccagatgat 840
tctcaaacta gatttgttga tattggaaaa gacttttatg catttcaaac gttcagtgag 900
gttgaacatg gagtcttagg tttagcttgg gcatcaaatt ggcagtatgc tgaccaggtt 960
ccgacaaatc catggagaag ttcaacgtcg ttagcaagaa attacacctt aagatatgtc 1020
catacaaatg ctgaaactaa acagctaaca ttgattcaaa atccagtttt accagattct 1080
atcaatgttg tagataaatt gaaaaagaaa aatgtcaaac ttaccaataa gaagccaatt 1140
aaaacaaatt tcaagggttc aacgggatta tttgatttta atattacatt taaagtatta 1200
aacttgaatg tgtctcctgg aaaaactcat tttgacattt taattaattc tcaagagttg 1260
aattcatcag ttgattccat taaaattggt tttgattcat cccagtcatc gttttatatc 1320
gatcgtcata ttccaaatgt tgaatttccc cgtaagcaat tctttactga taagttggct 1380
gcataccttg aacctttaga ctacgatcaa gacttaaggg tttttagttt gtatggtatt 1440
gttgacaaga atataattga gttgtatttt aatgacggaa cagttgctat gactaacaca 1500
ttcttcatgg gtgaaggtaa gtatccacac gatatacaaa ttgtgaccga tactgaagag 1560
ccgttatttg agttagagtc tgttatcata agggaactaa ataagtag 1608

## Description

The present invention is comprised within the biotechnology industry field and, in particular, in the food and agriculture sector dedicated to obtaining prebiotic oligosaccharides to be used as functional ingredients in dietary products, dairy products, infant foods and foods for animals. It also relates to the pharmaceutical and cosmetic industry field.

### State of the Art

Carbohydrates, the most abundant biological material in nature, are used as starting elements in a great variety of industrial processes; therefore, the enzymes involved in their metabolism have an enormous interest, both from a basic and technological point of view.

The field of prebiotic oligosaccharides as functional ingredients in food has developed spectacularly in the last few years.

The term prebiotic was introduced by Gibson and Roberfroid, who defined prebiotics as non-digestible ingredients of foods which beneficially affect the host by a selective stimulation of the growth and/or activity of one or a limited group of bacteria in the colon.

The leading prebiotic molecules on the European market are fructooligosaccharides (FOS) *(*P.T. Sangeetha, M.N. Armes, S.G. Prapulla, "Recent trends in the microbial production, analysis and application of fructooligosaccharides, Trends Food Sci. Technol. 2005, vol. 16, pg. 442-457*).* There are 3 types of fructooligosaccharides described up until now. The most known fructooligosaccharides (¹F series) are those that are currently marketed, and are formed by fructose molecules bound by β,2-1 bonds, with a glucose molecule at one end (J.W. Yun, "Fructooligosaccharides: Occurrence, preparation and application" Enzyme Microb. Technol. 1996, vol. 19, pp. 107-117), abbreviated as GFₙ, n typically being comprised between 2 and 4 (kestose, nystose and fructosylnystose). Fructooligosaccharides of the second type (⁶F series), in which the fructose molecules are bound by β,2-6 bonds, with a glucose molecule at the non-reducing end, are being intensively investigated. These FOS are normally found in nature in the form of high molecular weight polymers (levans). Neokestose, a trisaccharide in which a fructose is bound by a β,2-6 bond to the glucose unit in sucrose, is the first representative of the ⁶G series (the third type of FOS). The three types of FOS resist digestion in the upper part of the intestinal tract and are metabolized by the endogenous bacteria of the colon. It has been shown that they have the capacity to stimulate the growth of bifidobacteria (A.V. Rao, "Dose-response effects of inulin and oligofructose on intestinal bifidogenic effects" J. Nutr. 1998, vol. 80, pp. 1442S-1445S). FOS can be obtained by partial hydrolysis of natural polysaccharides (inulin, levan, etc.) or by enzymatic synthesis from sucrose. The profile of products (especially as regards the mean degree of polymerization) which is generated by both methodologies is considerably different, which has an impact on their properties.

The production of fructooligosaccharides of the ¹F series by a hydrolytic route from inulin or by a synthetic route by means of using sucrose 1-fructosyltransferases (EC 2.4.1.9.), β-fructofuranosidases -invertases- (EC 3.2.1.26) and even by levansucrases (EC 2.4.1.10) (L.E. Trujillo et al., "Fructo-oligosaccharides production by the Gluconacetobacter diazotrophicus levansucrase expressed in the methylotrophic yeast Pichia pastoris", Enzyme Microb. Technol. 2001, vol. 28, pp. 139-144) has been described. The synthesis yield that is reached using some invertases does not exceed 4% of the total oligosaccharides, whereas with 1-fructosyltransferases the yield that can be reached is much higher (around 50-60%) (M. Antosova, M. Polakovic, "Fructosyltransferases: the enzymes catalyzing production of fructooligosaccharides" Chem. Pap. 2001, vol. 55, pp. 350-358). FOS of the ¹F series are currently industrially produced using, in synthetic function, an *Aspergillus niger* β-fructofuranosidase for generating short-chain oligosaccharides with 3-5 units (C. Vannieeuwenburgh et al., "Kinetic studies and mathematical model for sucrose conversion by Aspergillus niger fructosyl-transferase under high hydrostatic pressure", Bioprocess Biosyst Eng. 2002, vol. 25, pp. 13-20). Other enzymes with transfructosidase activity have been described in fungi as *Aureobasidium pullulans, Aspergillus oryzae or Aspergillus japonicus* (C.S. Chien et al., "Immobilization of Aspergillus japonicus by entrapping cells in gluten for production of fructooligosaccharides". Enzyme Microb. Technol. 2001, vol. 29, pp. 252-257).

Low molecular weight fructooligosaccharides of the ⁶F series are obtained by means of acid hydrolysis from the levan polymer. By a synthetic route, only the formation of FOS of the ⁶F series as a minority product in the formation of 1-kestose from the *Zymomonas mobilis* levansucrase has been described (M. Bekers et al., "Fructooligosaccharide and levan producing activity of Zymomonas mobilis extracellular levansucrase", Process Biochem. 2002, vol. 38, 701-706). In the works referred to up until the now in the literature, whole cells of the yeast *Xanthophyllomyces dendrorhous* (also called *Phaffia rhodozyma*) or of several fungi (for example, *Penicillium citrinum,* immobilized cells) (Lee et al., "Reaction route for enzymatic production of neofructo-oligosaccharides from sucrose using Penicillium citrinum cells" J. Microbiol. 2001, vol. 39, pp. 331-333; Park et al., "Continuous production of neo-fructooligosaccharides by immobilization of whole cells of Penicillium citrinum", Biotechnol. Lett. 2005, vol. 27, pp. 127-130) are used for the production of fructooligosaccharides of the ⁶G series. The patent has been filed for an extracellular *X. dendrorhous* α-glucosidase with high-spectrum transglycosylase activity capable of generating malto- and isooligosaccharides from maltose (P200402994, UAM-CSIC) and for an extracellular fructofuranosidase of this yeast with transfructosidase activity capable of forming fructooligosaccharides of the ⁶G series (in particular neokestose) and ¹F (in particular 1-kestose) (P200501875, UAM-CSIC).

The effects of FOS on the person who consumes them can be very varied: reduction of diarrhea episodes caused by rotaviruses; improvement of lactose intolerance symptoms; control of constipation by increasing the fecal mass; increase of calcium absorption, and consequently a reduction of the risk of osteoporosis; decrease of the mutagenic capacity of certain microbial enzymes such as nitro-reductase, associated to colon cancer; possible reduction of diseases related to dyslipidemias, etc.

The yeast *Schwanniomyces occidentalis* (also *Debaryomyces occidentalis*) is capable of using a wide variety of organic compounds as a carbon source (glucose, fructose, galactose, sucrose, raffinose, lactose, maltose, citrate, ethanol, pullulan, dextrins, etc.). In biotechnology, this organism has been used because it shows an extremely efficient amylolytic system, which allows it to grow in media based on starch (USP 4794175). S. *occidentalis* expresses and secretes an invertase activity in media based on lactose (P. Costaglioli et al., "Secretion of invertase from Schwanniomyces occidentalis", Biotechnology Letters, 1997, vol. 19, pp 623-7). The enzyme was purified using a dual FPLC system based on Superose 12 and Mono Q (R.D. Klein et al., "Purification and characterization of invertase from a novel industrial yeast, Schwanniomyces occidentalis", Prep. Biochem, 1989, vol. 19, pp 293-319) and the sequenced encoding gene (Klein et al., "Cloning and sequence analysis of the gene encoding invertase from the yeast Schwanniomyces occidentalis", Curr Genet, 1989, vol. 16, pp 145-52*; sequence number X17604).* The amino acid sequence deduced from the nucleotide sequence has 533 amino acids (access number P24133). Nevertheless, no data relating to its activity on different substrates or on its fructosyltransferase capacity is available.

Given the industrial importance of prebiotic oligosaccharides, it is desirable to provide enzymes and methods for obtaining them which are industrially viable.

### Description of the Invention

The present invention relates to the characterization of a novel transfructosidase activity associated to the extracellular invertase of *Schwanniomyces occidentalis,* useful for obtaining prebiotic oligosaccharides, mainly 6-kestose. Thus, one aspect of the invention relates to a method for obtaining an enzymatic product with transfructosidase activity, which comprises culturing *S*. *occidentalis* cells in suitable medium and conditions. By means of conventional methods, the person skilled in the art will chose the culture media and the conditions such as pH, temperature and stirring for the culture of *S*. *occidentalis.* Culture examples are described in detail below.

Transfructosidase activity is associated to that of β-D-fructofuranosidase or invertase (EC 3.2.1.26) (IUBMB Enzyme Nomenclature, CAS Registry Number 9001-42-7), enzymes that hydrolyze sucrose into fructose and glucose.

The crude enzymatic product, result of the previous method of the invention, is ready to be used industrially for obtaining oligosaccharides without requiring subsequent separation or purification steps. In a particular embodiment of the invention, the method further comprises the step of recovering the enzymatic product from the culture medium and/or from the cells, since the enzyme object of the invention is extracellularly released. Thus, both the suspension of *S. occidentalis* cells with the suitable culture medium for which fructofuranosidase activity has been expressed, and the cell free fraction are understood as enzymatic product in this description. By means of conventional methods, the person skilled in the art will chose the starting enzymatic product most suited to each industrial process, that is, crude or with a higher or lower level of purification.

In another particular embodiment of the invention, the *S*. *occidentalis* cells belong to a strain selected from the group consisting of ATCC26077, ATCC26076, and ATCC20499. The nucleotide sequence of the gene encoding for the invertase (fructofuranosidase) activity shows remarkable differences with the sequence previously published and deposited in the data bank.

Another aspect of the invention relates to the enzymatic product with transfructosidase activity obtainable by the previously defined method. The enzymatic product of the invention is very efficient in sucrose degradation and also acts on oligosaccharides such as 1-kestose, nystose and raffinose. In a particular embodiment of the invention, the enzymatic product of the invention is characterized in that the fructofuranosidase activity has low substrate specificity, acting on sucrose, 1-kestose, nystose, and raffinose. In another particular embodiment, the fructofuranosidase activity of the enzymatic product presents a maximum in the pH range between 5 and 6 at 50°C, and in a temperature range between 40 and 55°C.

In addition to the fructofuranosidase activity, the enzymatic product of the invention has transfructosidase activity. In a particular embodiment, the products resulting from the transfructosidase activity are fructooligosaccharides of the ⁶F series (in particular 6-kestose) and as a minority, of the ¹F series (in particular 1-kestose). Another aspect of the invention relates to a method for obtaining a substantially pure enzyme with fructofuranosidase/transfructosidase activity, comprising the steps of: (a) obtaining an enzymatic product with fructofuranosidase/transfructosidase activity by means of the culture of *S*. *occidentalis* cells in suitable medium and conditions; (b) recovering the enzymatic product from the culture medium and/or from the cells; and (c) purifying the enzymatic product until obtaining a substantially pure enzyme with fructofuranosidase/transfructosidase activity.

The invention also relates to a substantially pure enzyme with fructofuranosidase/transfructosidase activity obtainable by the defined method. Conventional purification methods can be used for obtaining the enzyme of the invention. An example of a purification method is described in detail in this description.

The indicated substrate specificity characteristics for the enzymatic product of the invention are also attributed to the purified enzyme. Transfructosidase activity is also characteristic of the purified enzyme.

Some positive aspects of the enzymatic product and of the enzyme of the invention are that they have a varied action spectrum and a high specific activity, making them suitable candidates for hydrolyzing or modifying oligosaccharides. Another important aspect at the industrial level is that the enzyme is stable during long reaction times (for example, 144 h) at a temperature of approximately 50°C in the presence of high sucrose concentrations.

The present invention entails a method for obtaining industrially viable fructooligosaccharides, mainly 6-kestose. Thus, another aspect of the invention relates to a method for obtaining oligosaccharides which comprises allowing the previously defined enzymatic product or purified enzyme to act on one or several glucidic substrates. By means of conventional methods, the person skilled in the art will chose the culture media, the substrates and the reaction conditions for carrying out the method. In addition, the enzyme or the *S*. *occidentalis* cells, producers of the enzyme, can be used as such or in an immobilized manner, physically or chemically coupled to a carrier material. The reuse of the enzyme or the cells is thus allowed. Preparation examples are included below in this description.

### ENZYMATIC CHARACTERIZATION

### A) Expression of the fructofuranosidase activity of S. occidentalis.

The production of fructofuranosidase activity was analyzed in cultures of S. *occidentalis* of the ATCC26077, ATCC26076, and ATCC20499 strains, grown in medium for yeasts supplemented with lactose. The cultures were carried out in glass flasks incubated at a temperature comprised between 28-30°C and with constant orbital stirring of 180-235 rpm. The optimal growth conditions were 29°C and 235 rpm.

The cell free fraction was obtained by centrifugation (F-0) of a culture of S. *occidentalis* ATCC26077 grown at 29°C and 235 rpm. Fructofuranosidase activity was assayed in the latter, determining the glucose release on different substrates. A colorimetric assay and the standard methodology were used. The released glucose was quantified using the glucose oxidase-peroxidase coupled reaction: 0.4 ml of the solution to be determined was mixed with 0.1 ml of A:B (20:1) solution (A: 0.85 U/ml glucose oxidase, 0.40 U/ml peroxidase in sodium phosphate buffer pH 5; B: 0.6% O-dianisidine). It was incubated for 30 minutes at 37 °C and spectrophotometrically quantified at 450 nm. A standard glucose curve (0 to 100 µg/ml) was used. The fructofuranosidase activity unit is defined in µmol/ml min of glucose determined in the described conditions. Figure 1 shows the obtained results when the ATCC26077 strain is used and the assay is performed on sucrose.

### B) Characterization of the fructofuranosidase activity of S. occidentalis after the culture and centrifugation of the cell free fraction.

The cell free fraction, obtained by centrifugation, of a culture of *S*. *occidentalis* grown in lactose medium: 0.3% (w/v) YNB (yeast nitrogen base w/o amino acids, DIFCO), 3.5% (w/v) bactopeptone (DIFCO), 0.5% (w/v) KH₂PO₄, 1% (w/v) MgSO₄·7H₂O, 1% (w/v) (NH₄)SO₄, and 2% lactose as a carbon source (R.D. Klein et al., "Purification and characterization of invertase from to novel industrial yeast, Schwanniomyces occidentalis", Prep. Biochem, 1989, vol. 19, pp. 293-319), grown until an optical density of 8.34 OD 660 nm, was concentrated using a tangential filtration system (30 kDa filter) followed by dialysis against 20 mM HCl-Tris pH 7 for 2 hours at a temperature of 4°C and was assayed on different substrates. The assay results are shown in Table 1. No activity was detected when lactose, leucrose, turanose or palatinose were used as substrate.

| TABLE 1. Fructofuranosidase activity of the extracellular fraction of *S*. *occidentalis* on different carbohydrates. | |
|---|---|
| Substrate | Specific activity (mU/µg) |
| sucrose | 24 |
| 1-kestose | 2.2 |
| nystose | 0.6 |
| raffinose | 0.2 |

### C) Purification of the enzyme with fructofuranosidase activity of S. occidentalis.

For the purification of the enzyme, 2 liters of extracellular fraction of *S. occidentalis,* with a fructofuranosidase activity of 0.58 U/ml, were used to begin. The following method was used:
1) Concentration of the extracellular fraction using a tangential filtration system (30 kDa filter) followed by dialysis against 20 mM HCl-Tris pH 7 (buffer A) for 2 hours at a temperature of 4°C. 35 ml of concentrate with an activity of 23.28 U/ml (F-1) were obtained.
2) Ion exchange chromatography at pH 7. 10 ml of the sample were applied to a 20 ml DEAE-Sephacel ion exchange column equilibrated with buffer A. The elution was carried out using a NaCl gradient of 0 to 2 M. The fraction eluted at 0.2 M of salt showed an activity of 3.30 U/ml. It was dialyzed against 20 mM sodium acetate pH 5 (buffer B) for 2 hours at 4°C (F-2).
3) Ion exchange chromatography to pH 5. The sample was applied to the ion exchange DEAE-Sephacel column equilibrated with buffer B and was eluted using a discontinuous NaCl gradient of 0.05, 0.1, 0.15, 0.2, 0.25, 0.3, 0.4 and 0.5 M of salt. The fructofuranosidase activity determined as previously indicated eluted at 0.2 M of salt and was 0.33 U (F-3).

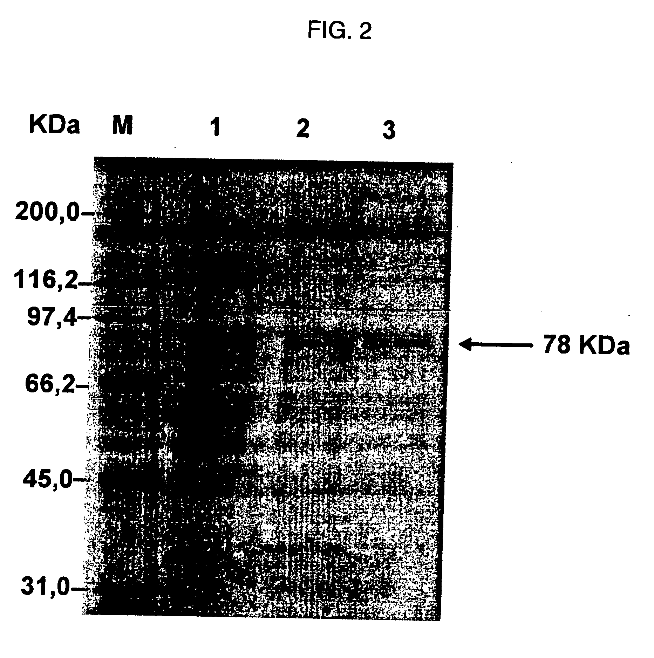

The purification was determined by analyzing the proteins present after each of the purification steps (F-1, F-2 and F-3) in SDS-PAGE polyacrylamide gels and staining with Coomassie blue. The obtained results are shown in Figure 2.

### D) Characterization of the fructofuranosidase activity of the purified enzyme.

The hydrolytic activity of the purified enzyme (F-3), following the described method, was assayed on different substrates. The maximum activity level is obtained on sucrose. Activity on lactose, leucrose, turanose or palatinose was not observed. The obtained results are compiled in Table 2.

| TABLE 2. Fructofuranosidase activity of the F-3 fraction of *S. occidentalis* on different substrates. The assays were performed using purified protein and a concentration of 1% for all assayed substrates. | |
|---|---|
| Substrate | Activity specific (mU/µg) |
| sucrose | 109 |
| 1-kestose | 6.3 |
| raffinose | 7.5 |
| nystose | 6.2 |

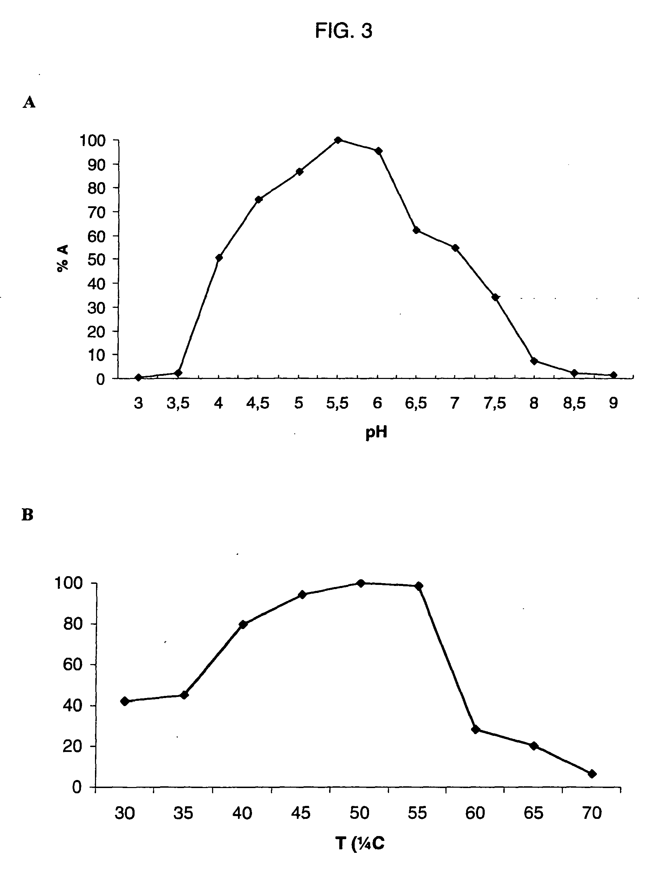

The fructofuranosidase activity was assayed at different pH and temperatures. Maximum activity levels were obtained in a pH range comprised between 5 and 6 units at 50°C and at a temperature of 40 and 55°C. Figure 3 shows the obtained results.

### E) Characterization of the gene encoding for the enzyme with fructofuranosidase activity of S. occidentalis.

The encoding gene (1.6 kb) of the fructofuranosidase activity of *S. occidentalis* ATCC26077, ATCC26076, and ATCC20499 was amplified using the PCR standard technique (Polymerase Chain Reaction), genomic DNA of the yeast and oligonucleotides directed towards the sequence of the ends of the open reading frame (ORF) of the gene, already deposited in the databases (*sequence of access number X17604*).

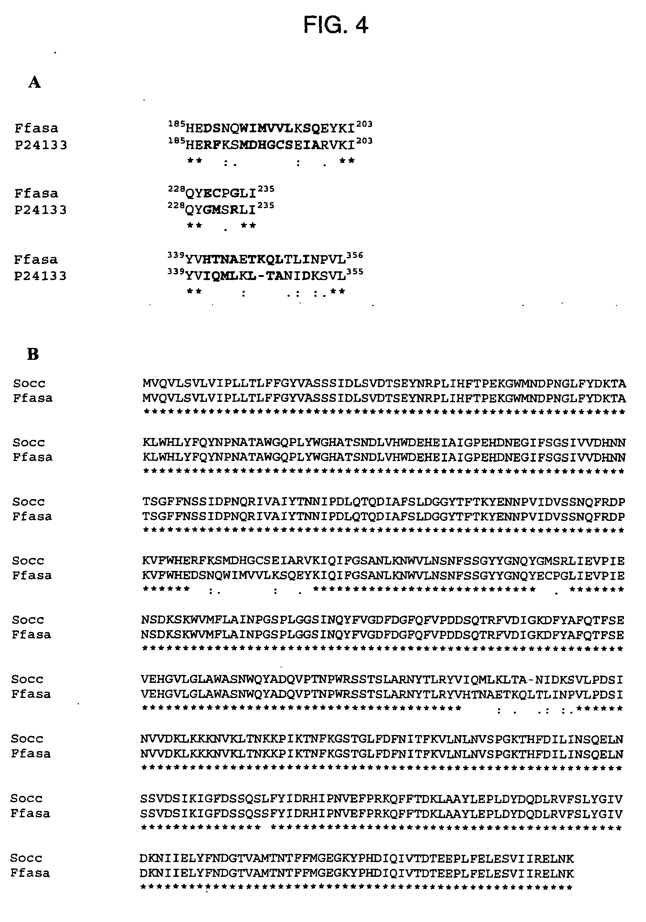

The sequence of the amplified genetic material in the three strains of yeast analyzed has remarkable differences with respect to the previously published nucleotide sequence. The deduced protein with its identification sequence SEQ. ID. NO: 1 has 535 amino acids instead of the 533 previously described and has four sequences, of 15, 4, 14 and 1 amino acid, different from those published. Figure 4 shows the changes that were obtained when comparing the amino acid sequence of the fructofuranosidase (invertase) deposited in the data banks *(P24133)* and the one obtained in this work.

### F) Transfructosidase activity of the F-1 fraction of S. occidentalis

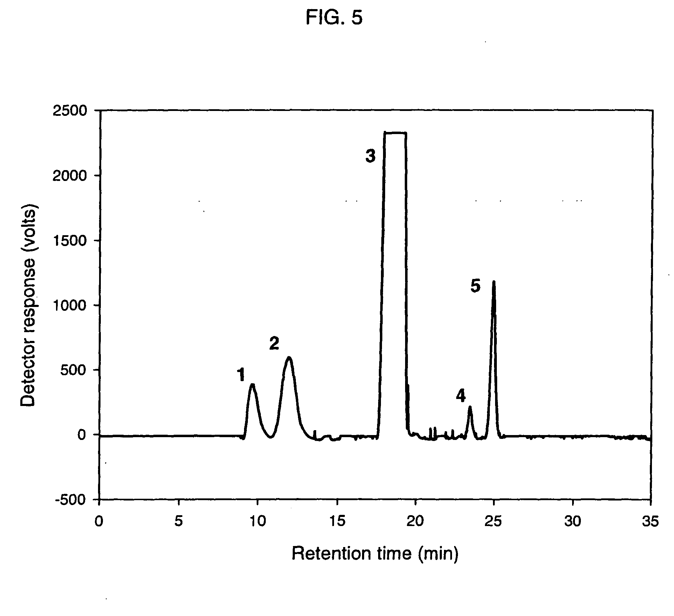

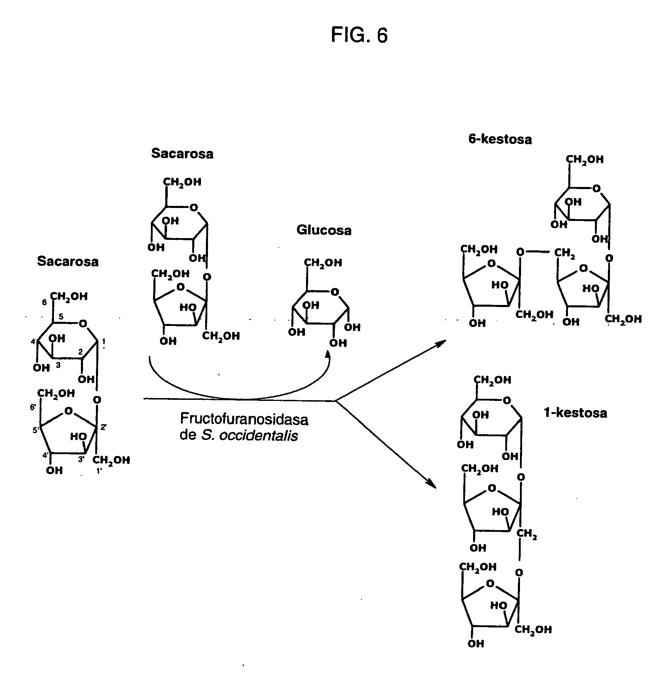

The transglycosylation activity of the enzymatic concentrate obtained by ultrafiltration (F-1) was assayed. A reaction was prepared using a high concentration of sucrose (510 g/i), to favor the formation of glycosidic bonds in detriment of the hydrolysis reaction, and a final activity enzymatic in the reaction mixture of approximately 5 U/ml. Figure 5 shows the chromatogram of the reaction mixture at 6 hours. It can be seen that the enzyme of *S. occidentalis* jointly shows hydrolysis activity and transfer activity. On one hand, fructose (peak 1) and glucose (peak 2) are formed as hydrolytic products. On the other hand, two trisaccharides are obtained: a majority trisaccharide (peak 4), identified as 6-kestose [β-D-Fru-(2→6)-β-D-Fru-(2→1)-α-D-Glu] and another minority trisaccharide (peak 5), identified as 1-kestose [β-D-Fru-(2→1)-β-D-Fru-(2→1)-α-D-Glu]. The sucrose that has not reacted corresponds to peak 3. The scheme of the transglycosylation reaction is shown in Figure 6.

Table 3 shows the composition (in g/I) of the carbohydrates present in the reaction mixture over 24 hours of incubation at 50°C. It is observed that the 6-kestose/1-kestose molar ratio reaches a maximum value of 3.3 between 4 and 6 hours of reaction. In the maximum point of FOS production (4 hours), 43.6 g/I of FOS, which corresponds to a percentage of 8.6% of FOS with respect to the total carbohydrates in the mixture, were obtained.

| TABLE 3. Composition of the reaction mixture over time after incubation at 50°C of sucrose with the enzymatic concentrate, F1, of ***S.**occidentalis.* Reaction conditions: 600 g sucrose/I in 0.2 M sodium acetate (pH 5.6), 150 rpm. The assay was performed with 5 U/ml of biocatalyst. Analysis by means of HPLC using a Waters delta 500 pump, 250 x 4.6 mm Lichrospher 100-NH2 column (Merck), acetonitrile:water 75:25 v/v, 0.7 ml/min, 25°C, light-scattering evaporative detector. Compound names: 1, fructose; 2, glucose; 3, sucrose [α-D-Glu-(1→2)-β-D-Fru]; 4, 1-kestose [β-D-Fru-(2→1)-β-D-Fru-(2→1)-α-D-Glu]; 5, 6-kestose [βD-Fru-(2→)-βD-Fru-(2→)-αD-Glu] | | | | | | |
|---|---|---|---|---|---|---|
| **Reaction time (h)** | **Composition of the reaction mixture (grams/liter)** | | | | | **Percentage (w/w) of FOS**^{a} |
| | **1** | **2** | **3** | **4** | **5** | |
| 0 | 0 | 0 | 510 | 0 | 0 | 0 |
| 1 | 5.3 | 9.2 | 487.3 | 2.8 | 5.4 | 1.6 |
| 1.5 | 16.8 | 20.4 | 453.8 | 6.4 | 12.7 | 3.7 |
| 4 | 44.3 | 60.5 | 361.6 | 10.1 | 33.5 | 8.6 |
| 6 | 54.8 | 70.9 | 343.7 | 9.6 | 31.0 | 7.9 |
| 24 | 113.6 | 140.3 | 240.9 | 4.5 | 10.6 | 3.0 |
| ^{a} Percentage of fructooligosaccharides with respect to the total weight of sugars. | | | | | | |

### G) Transfructosidase activity of the fraction F-3 of S. occidentalis

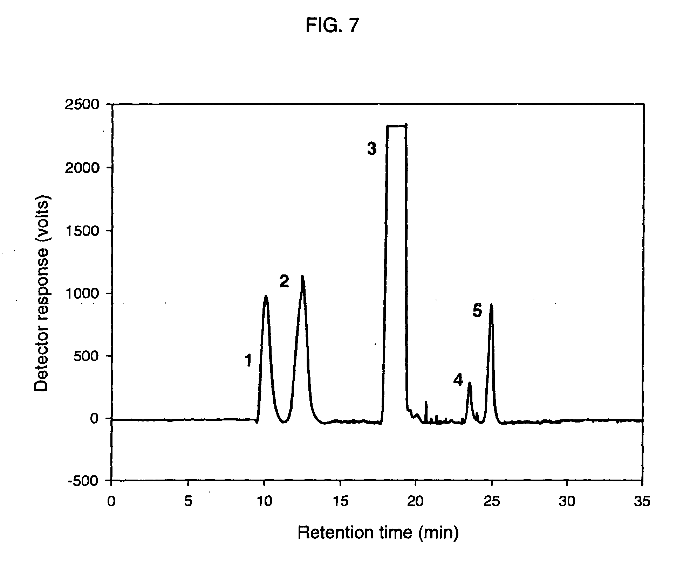

The transglycosylation activity of the pure enzyme (fraction F-3) was assayed. A reaction was prepared using a high concentration of sucrose (510 g/I) and a final enzymatic activity in the reaction mixture of approximately 0.5 U/ml. Figure 7 shows the chromatogram of the reaction mixture at 144 hours. It is seen that the profile of the obtained products is very similar in both cases, specifically the 6-kestose/1-kestose ratio that was obtained was 3.3.

The novel enzyme characterized in this work still maintains transfructosidase activity after 144 hours at 50°C. After 144 hours of reaction, 42.6 g/I of FOS were obtained. The total percentage (w/w) of fructooligosaccharides was 8.4%, this value being with respect to the total weight of carbohydrates in the medium.

Throughout the description and the claims, the word "comprises" and its variants do not intend to exclude other technical characteristics, additives, components or steps. For the persons skilled in the art, other objects, advantages and features of the invention will be understood in part from the description and in part from the practice of the invention. The following detailed description, examples and drawings are provided by way of illustration and do not intend to limit the present invention

### Description of the Drawings

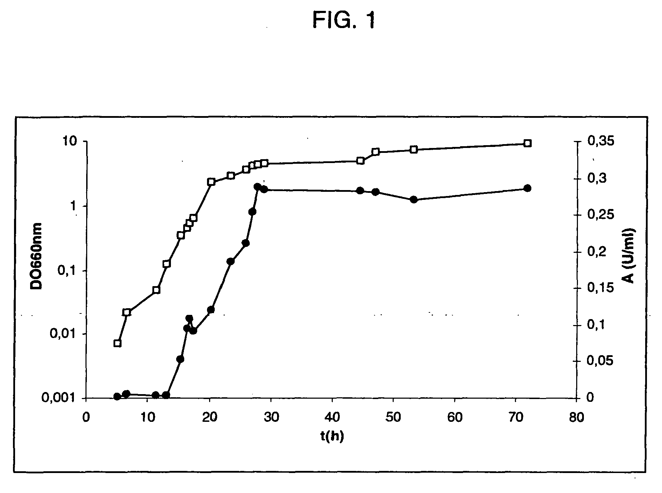

Figure 1 shows the production of extracellular fructofuranosidase activity throughout the culture of *S. occidentalis* ATCC26077. The yeast was grown in 2% lactose medium at 29°C and constant orbital stirring of 235 rpm for 72 hours. The growth of the culture is represented in OD 660 nm (squares) and the fructofuranosidase activity determined in the extracellular medium in U/ml (circles), reached at the times that are indicated. The activity was assayed on 0.5% sucrose.
Figure 2 shows the result of the analysis by SDS-PAGE (8%) of the proteins present throughout the purification process. 1 ml of the indicated fractions was precipitated with TCA (10% (w/v)), resuspended in 10 µ of 1.5 M HCl-Tris pH 7.5 and analyzed in 8% polyacrylamide gels. The gel was stained with Coomassie blue following the standard methodology. M: molecular weight markers; 1, 2 and 3: F-1, F-2 and F-3, respectively.
Figure 3 indicates the enzymatic activity in function of the pH and of the temperature, showing the corresponding maximums. The assays were performed on sucrose using a solution of pure protein. 100% corresponds to an activity of 5.59 U/ml.
   Figure 3A: fructofuranosidase activity was determined at different pHs (3-9 pH units), an assay temperature of 50°C and sodium citrate, sodium phosphate and HCl-Tris buffer (all of them 100 mM) for the pH range of 3-4.5, 5.0-7.5 and 8.0-9.0, respectively.
   Figure 3B: the temperature assay (30-70°C) was performed in 50 mM sodium phosphate pH 5.5.
Figure 4 is the result of the analysis of the sequence of the fructofuranosidase of S. *occidentalis* ATCC26077 obtained in this work. The amino acid sequence of the protein previously described *(P24133)* and the one obtained in this work (Ffase) is shown.
Figure 5 shows the profile of products obtained after the incubation of sucrose at a concentration of 510 g/l with the enzymatic product (enzymatic concentrate F-1) of *S. occidentalis.* The reaction conditions, the HPLC analysis conditions and the compound names are the same as those in Table 3. The chromatographic analysis corresponds to 6 hours of reaction.
Figure 6 shows a scheme of the transglycosylation reaction catalyzed by the fructofuranosidase of *S. occidentalis.*
Figure 7 shows the profile of products obtained after the incubation of sucrose at a concentration of 510 g/I with the pure enzyme (F-3) of *S. occidentalis.* The reaction conditions, the HPLC analysis conditions and the compound names are the same as those in Table 3. The chromatographic analysis corresponds to 144 hours of reaction.

### EXAMPLES

### EXAMPLE 1: Production of fructofuranosidase throughout cultures of S. occidentalis ATCC26077 grown in minimum media.

For the production of fructofuranosidase, *S. occidentalis* of the ATCC26077 strain was cultured in 100 ml of minimal medium for yeasts supplemented with lactose (MML), sucrose (MMS), raffinose (MMR) and glucose (MMG): 0.7% (w/v) YNB (Yeast Nitrogen Base w/o Amino acids, DIFCO), and the corresponding carbon source in the following percentages (w/v): 2% lactose, 0,5% sucrose, 0.2% raffinose and 0.05% glucose. The cultures were kept for 40 hours. The cell growth was spectrophotometrically determined following the absorbance of the culture at an optical density of 660 nm (OD 660). 250 ml glass flasks, temperature of 29 °C and constant orbital stirring of 230 rpm were used. The stationary phase was reached at 0.112, 0.757, 0.455 and 0.160 OD 660, for the media based on lactose, sucrose, raffinose and glucose, respectively.

### EXAMPLE 2: Use of the enzyme for glucose production from sucrose with minimal medium supernatant.

The supernatants of the cultures of Example 1 were used for releasing glucose from sucrose by action of the fructofuranosidase activity of the supernatant where the extracellular enzyme is found. To that end, 100 µl of the cell free fraction, 0.4 ml of 50 mM sodium phosphate buffer pH 5.5 and 0.5 ml of 1 % sucrose (w/v) in this same buffer were mixed and incubated at 42°C for 90 minutes. Fructofuranosidase activity was observed from the beginning of the logarithmic growth phase of the cultures until the end of the stationary phase. The maximum activity levels (107 mU/ml for MML, 9.6 mU/ml for MMG, 0.78 mU/ml for MMS and 1.2 mU/ml for MMR) were obtained in the stationary phase.

### EXAMPLE 3: Production of fructofuranosidase throughout cultures of S. occidentalis grown in medium rich with raffinose.

For the production of fructofuranosidase, *S. occidentalis* ATCC 26077 was cultured in 100 ml of medium (YEP) supplemented with raffinose (YEPR): 1% (w/v) yeast extract (DIFCO), 2% (w/v) bactopeptone (DIFCO), 0.2% (w/v) raffinose. A 250 ml glass flask, temperature of 30°C and constant orbital stirring of 200 rpm were used. The culture was kept for 48 hours. Cell growth was carried out and determined as in the previous example. The stationary phase was reached at 28 hours of growth, to 2.28 OD 660.

### EXAMPLE 4: Use of the enzyme for the glucose production from sucrose with supernatant of medium with raffinose.

The supernatant of the culture of Example 3 was used for releasing glucose from sucrose by action of the fructofuranosidase activity of the supernatant where the extracellular enzyme is found as in Example 2. Levels of fructofuranosidase activity that could be determined were obtained from midway through the logarithmic growth phase of the culture until the end of the curve, for about 30 hours of culture. The maximum activity levels (0.382 U/ml) were obtained at 2.28 OD 660.

### EXAMPLE 5: Stability of the fructofuranosidase of S. occidentalis at 50 and 60°C.

The extracellular fraction of a culture of *S. occidentalis* was concentrated 57 times using a tangential filtration system (30 kDa filter) followed by dialysis against 20 mM sodium phosphate pH 7 for 2 hours at a temperature of 4°C. An enzymatic preparation with an activity of 23.3 U/ml was obtained. The preparation was maintained at 50 and 60°C for different times. 50 µl of the preparation were taken every 5-10 minutes. The fructofuranosidase activity on sucrose was determined in all the samples obtained as in Example 2 (50°C, 20 min). After 45 and 120 minutes at 50°C, 50 and 20% of the initial activity, respectively, are maintained. However after 2 and 5 minutes at 60°C, only 80 and 25% of the activity, respectively, are maintained.

### EXAMPLE 6: Obtaining the genomic DNA of S. occidentalis.

The ATCC20499 strain of *S. occidentalis* is cultured in minimal medium for yeasts supplemented with 2% lactose (MML) at 29°C with constant orbital stirring of 235 rpm, until an optical density of 7.58 OD 660 nm. The genomic DNA was isolated basically following a previously described genomic DNA extraction protocol for Southern blot analysis (M.D. Rose et al. Methods in yeast genetics to laboratory course manual. Cold Spring Harbor Laboratory Press. 1990), resuspended in 50 µl of 10 mM HCl-Tris pH 8.5 and analyzed in agar gel (0.7% w/v). The final concentration of DNA was 100 ng/µl.

### EXAMPLE 7: Amplification of the fructofuranosidase gene by means of the PCR (Polymerase Chain Reaction) technique.

The DNA obtained according to the method described in Example 6 was used as a mold for amplifying the fructofuranosidase (invertase) gene (X17604) of *S. occidentalis.* The PCR technique was used. To that end, two oligonucleotides primers were designed with the SEQ. ID. NO: 2 and SEQ. ID. NO: 3 sequences specific for the sequence of the gene X17604 and which include the *Bam*HI and *Xho*I restriction sequences, respectively. Each reaction includes: 1.25 U of TaqPol (Promega), 2.5 µl of the buffer for this enzyme 10x, 2.5 µl of 25 mM MgCl₂, 0.25 µl of 40 mM dNTs, 5 µl of the genomic DNA obtained as indicated in Example 5, 1.5 µl of each of the oligonucleotides primers and H₂O until a final volume of 25 µl. This reaction mixture was incubated: a) 10 minutes at 94°C, b) 3 cycles of 94°C 1 minute, 57°C 1 minute and 72°C 1 minute, c) 35 cycles of 94°C 1 minute, 57°C 1 minute and 72°C 1.5 minutes and d) 1 cycle of 94°C 1 minute, 57°C 1 minute and 72°C 6 minutes. The product was analyzed in agar gel. A 1.6 kb fragment, which corresponds to the expected size for the gene X17604, at a concentration of about 50 ng/µl was amplified.

### EXAMPLE 8: Cloning of the product amplified by PCR and sequencing of the same.

The PCR product obtained as indicated in Example 7 was purified by means of QIAEX II Gel Extraction Kit 150 of QIAGEN, and included in the pSTBlue-1 vector of Novagen (Perfectly Blunt cloning) linearized with EcoRV. *E. coli* DH5α competent for transformation by heat shock were used and the selection was performed in LB with Ampicillin (100 µg/ml), IPTG and X-Gal. The plasmid DNA was isolated using the kit: DNA Purification System, Wizard Plus SV System of Promega and the construct was analyzed by sequencing. The obtained sequence was compared to the one described, access number X17604. Taking the genetic code into account, the amino acid sequence for the encoded protein was obtained, which was made up of 535 amino acids, one amino acid more than that described in access sequence P24133. In addition, the sequence of the novel enzymatic activity differs in four areas with the previously published sequence (P24133). The differences are shown in Figure 4.

### EXAMPLE 9: Formation of fructooligosaccharides at 50°C from sucrose catalyzed by the enzymatic concentrate (F-1) of S. occidentalis.

A high concentration solution of sucrose (510 g/I) in 0.2 M sodium acetate buffer pH 5.6 was prepared. The fructofuranosidase was added until a final concentration in the reaction mixture of 5 U/ml (one unit U is the enzymatic activity corresponding to release of a micromole of reducing sugars per minute, using 100 g/l of sucrose as substrate, in 0.2 M sodium acetate buffer pH 5.6, at 50°C). The reaction mixture was incubated for 24 hours at 50°C, with orbital stirring at 700 rpm. Aliquots were extracted at different times, incubated for 5 minutes at 80°C to inactivate the enzyme, diluted 1:2 (v/v) with water, centrifuged for 5 minutes at 6000 rpm in an eppendorf tube with a 0.45 µm filter and analyzed by HPLC liquid chromatography. The profile of the products formed is seen in Figure 5. It is observed that the fructofuranosidase of *S. occidentalis* has transfer activity (transfructosylation). Two trisaccharides are obtained: one majority trisaccharide, 6-kestose β-D-Fru-(2→6)-β-D-Fru-(2→1)-α-D-Glu] and another minority trisaccharide, identified as 1-kestose [β-D-Fru-(2→1)-β-D-Fru-(2→1)-α-D-Glu]. In the maximum point of FOS production (4 h), the composition of the system was: 8.7% fructose, 11.9% glucose, 70.8% sucrose, 2.0% 1-kestose and 6.6% 6-kestose.

### EXAMPLE 10: Formation of fructooligosaccharides at 50°C from sucrose catalyzed by the pure enzyme (F-3) of S. occidentalis.

A high concentration solution of sucrose (510 g/I) in 0.2 M sodium acetate buffer pH 5.6 was prepared. The fructofuranosidase was added until a final concentration in the reaction mixture of 0.5 U/ml (one unit U is the enzymatic activity corresponding to releasing a micromole of reducing sugars per minute, using 100 g/I of sucrose as substrate, in 0.2 M sodium acetate buffer pH 5.6, at 50°C). The reaction mixture was incubated for 144 hours at 50°C, with orbital stirring at 700 rpm. Aliquots were extracted at different times, incubated for 5 minutes at 80°C to inactivate the enzyme, diluted 1:2 (v/v) with water, centrifuged for 5 minutes at 6000 rpm in an eppendorf tube with a 0.45 µm filter and analyzed by HPLC liquid chromatography. The profile of the products formed is seen in Figure 7. Two trisaccharides were obtained: a majority trisaccharide, identified as 6-kestose, and another minority trisaccharide, identified as 1-kestose. At the final reaction time (144 h), the composition of the system was: 12.7% fructose, 14.8% glucose, 64.1 % sucrose, 2.4% 1-kestose and 5.9% 6-kestose).

## Claims

1. A method for obtaining an enzymatic product with fructofuranosidase activity, which comprises culturing *Schwanniomyces occidentalis* (also *Debaryomyces occidentalis*) cells in a medium for yeasts based on lactose, at a temperature of 29°C and constant orbital stirring of 235 rpm.

2. The method according to claim 1, which comprises culturing *Schwanniomyces occidentalis* cells in a minimal or rich medium for yeasts based on different carbon sources (lactose, sucrose, raffinose, glucose) at a temperature comprised between 28 and 30°C and a constant orbital stirring range comprised between 180 and 235 rpm.

3. The method according to claims 1 and 2, comprising the additional step of recovering the enzymatic product from the culture medium and/or from the cells.

4. The method according to any of claims 1 to 3, wherein the *Schwanniomyces occidentalis* cells belong to a strain selected from the group consisting of ATCC26077, ATCC26076 and ATCC20499.

5. An enzymatic product with fructofuranosidase activity obtainable by the method defined in any of claims 1 to 4.

6. The enzymatic product according to claim 5, **characterized in that** the fructofuranosidase activity has low substrate specificity, acting on sucrose, 1-kestose, nystose and raffinose.

7. The enzymatic product according to any of claims 5 and 6, **characterized in that** it has no fructofuranosidase activity on lactose, leucrose, turanose or palatinose.

8. The enzymatic product according to any of claims 5 to 7, wherein the fructofuranosidase activity has a maximum in the pH range between 5 and 6 units at 50°C, and in a temperature range from 40 to 55°C.

9. The enzymatic product according to any of claims 5 to 8, **characterized in that** it has transfructosidase activity in the presence of one or several glucidic substrates.

10. The enzymatic product according to claim 9, **characterized in that** the glucidic substrates are fructooligosaccharides.

11. The enzymatic product according to claim 10, wherein the products resulting from the transfructosidase activity are fructooligosaccharides with β-1,2, and β-2,6 bonds.

12. The enzymatic product according to claim 11, wherein the products resulting from the transfructosidase activity are basically 6-kestose and 1-kestose

13. A method for obtaining oligosaccharides which comprises allowing the enzymatic product defined in any of claims 5 to 12 to act on one or several glucidic substrates.

14. A method for obtaining a substantially pure enzyme with fructofuranosidase activity from the enzymatic product obtained by the method according to claims 1 and 2, comprising the additional step of purifying the enzymatic product until obtaining the substantially pure enzyme.

15. The method according to claim 14, wherein the *Schwanniomyces occidentalis* cells belong to a strain selected from the group consisting of ATCC26077, ATCC26076 and ATCC20499.

16. A substantially pure enzyme with fructofuranosidase activity obtainable by the method defined in any of claims 14 and 15.

17. The enzyme according to claim 16, **characterized in that** the fructofuranosidase activity has low substrate specificity, acting on sucrose, 1-kestose, nystose and raffinose.

18. The enzyme according to any of claims 16 and 17, **characterized in that** it has no fructofuranosidase activity on lactose, leucrose, turanose, palatinose,

19. The enzyme according to any of claims 16 to 18, wherein the fructofuranosidase activity has a maximum in the pH range between 5 and 6 at 50°C, and in a temperature range between 40 and 55°C.

20. The enzyme according to any of claims 16 to 19, **characterized in that** it has transfructosidase activity in the presence of one or several glucidic substrates.

21. The enzyme according to claim 20, **characterized in that** the glucidic substrates are fructooligosaccharides.

22. The enzyme according to claim 21, wherein the products resulting from the transfructosidase activity are oligosaccharides with β-1,2, and β-2,6 bonds.

23. The enzyme according to claim 22, wherein the products resulting from the transfructosidase activity are basically 6-kestose and 1-kestose.

24. The enzyme according to any of claims 16 to 23, **characterized by** its amino acid sequence SEQ. ID. NO: 1 and by having 535 amino acids.

25. A DNA sequence encoding the enzyme defined in any of claims 16 to 24, **characterized by** its nucleotide sequence SEQ. ID. NO: 4.

26. A method for obtaining oligosaccharides which comprises allowing the enzyme defined in any of claims 16 to 25 to act on one or several glucidic substrates.
